(19) Europäisches Patentamt
European Patent Office
Office européen des brevets

(11) **EP 3 426 143 B1**

(12) **EUROPÄISCHE PATENTSCHRIFT**

(45) Veröffentlichungstag und Bekanntmachung des Hinweises auf die Patenterteilung:
**13.01.2021  Patentblatt 2021/02**

(21) Anmeldenummer: **17707751.8**

(22) Anmeldetag: **21.02.2017**

(51) Int Cl.:
*A61B 5/048* (2006.01)     *A61B 5/0484* (2006.01)
*A61B 5/0478* (2006.01)    *A61N 1/04* (2006.01)
*A61N 1/05* (2006.01)      *A61N 1/372* (2006.01)
*A61N 1/36* (2006.01)

(86) Internationale Anmeldenummer:
**PCT/AT2017/060036**

(87) Internationale Veröffentlichungsnummer:
**WO 2017/152204 (14.09.2017 Gazette 2017/37)**

(54) **VORRICHTUNG ZUR ELEKTROSTIMULATION EINES PROBANDEN**

APPARATUS FOR ELECTROSTIMULATION OF A TEST SUBJECT

DISPOSITIF D'ÉLECTROSTIMULATION D'UN SUJET

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.03.2016  AT 501892016**

(43) Veröffentlichungstag der Anmeldung:
**16.01.2019  Patentblatt 2019/03**

(73) Patentinhaber:
• **Guger, Christoph**
  **4533 Piberbach (AT)**
• **Edlinger, Günter**
  **8020 Graz (AT)**

(72) Erfinder:
• **Guger, Christoph**
  **4533 Piberbach (AT)**
• **Edlinger, Günter**
  **8020 Graz (AT)**

(74) Vertreter: **Wildhack & Jellinek**
**Patentanwälte**
**Landstraßer Hauptstraße 50**
**1030 Wien (AT)**

(56) Entgegenhaltungen:
**US-A1- 2002 095 097     US-A1- 2002 147 411**
**US-A1- 2009 082 691     US-A1- 2011 264 165**
**US-A1- 2012 253 421**

• **WHEELER JESSE J ET AL: "An implantable 64-channel neural interface with reconfigurable recording and stimulation", 2015 37TH ANNUAL INTERNATIONAL CONFERENCE OF THE IEEE ENGINEERING IN MEDICINE AND BIOLOGY SOCIETY (EMBC), IEEE, 25. August 2015 (2015-08-25), Seiten 7837-7840, XP032812000, DOI: 10.1109/EMBC.2015.7320208 [gefunden am 2015-11-04]**

EP 3 426 143 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft eine Vorrichtung zur Elektrostimulation einer Person umfassend eine Anzahl von das an das menschliche Gehirn anlegbaren Elektroden.

Bei einer Vielzahl von medizinischen Anwendungen ist es erforderlich, einzelne Regionen des Gehirns sowie deren zugehörige Funktionen zu identifizieren. Insbesondere kann es für bestimmte Anwendungen von Vorteil sein, Regionen des Gehirns zu erkennen, in denen konkrete motorische, auditorische, visuelle, sensorische oder andere Abläufe gesteuert werden.

Aus dem Stand der Technik sind Elektrostimulationsvorrichtungen bekannt, die eine Vielzahl von Elektroden aufweisen, die unmittelbar an das menschliche Gehirn angelegt werden. Die US 2011/264165 A1 offenbart eine Vorrichtung zur Elektrostimulation eines Probanden mit einer Anzahl von in das Gehirn des Probanden implantierten Elektroden. Anhand der Messsignale zumindest einer Messelektrode und eines physiologischen Modells können Elektroden ausgewählt und bestimmte Regionen des Gewebes stimuliert werder Die US-2012/253421 -A1 offenbart eine weitere Stimulationsvorrichtung mit Vorauswahl der Stimulationselektroden.

Im Zuge der aus dem Stand der Technik bekannten Vorgehensweise werden Regionen des menschlichen Gehirns analysiert, indem eine große Anzahl von Elektroden an das menschliche Gehirn angelegt wird. Anschließend werden an einzelne benachbart gelegene Elektroden Stimuli in Form von Spannungen angelegt, wodurch Stimuli in Form von elektrischen Strömen durch das menschliche Gehirn fließen. Diese Anregung führt dazu, dass der Proband, an dessen Gehirn die Elektroden angelegt sind, bestimmte Wahrnehmungen/Gedanken hat oder bestimmte Körperbewegungen durchführt. Um mit der aus dem Stand der Technik bekannten Maßnahme die Position bestimmter Gehirnareale zu identifizieren, die bestimmte Funktionen erfüllen, ist es erforderlich, sämtliche an das Gehirn angelegte Elektroden zu aktivieren bzw. zu stimulieren und anschließend die Reaktion des Probanden abzuwarten. Insbesondere kann es erforderlich sein, den Stimulus im Lauf der Anwendung zu verstärken, um eine Reaktion beim Probanden herbeizuführen. Dieses Vorgehen ist äußerst aufwendig und zeitintensiv und hat darüber hinaus den Nachteil, dass bei Probanden, die zu epileptischen Anfällen neigen, vermehrt epileptische Anfälle ausgelöst werden. Weiters kann es bei Kindern oder Patienten schwierig sein, richtige Wahrnehmungsbeschreibungen zu erhalten.

Aufgabe der Erfindung ist es daher, eine Vorrichtung zur Verfügung zu stellen, die insgesamt mit einer geringeren Anzahl von Stimuli auf das menschliche Gehirn auskommt und dennoch eine vorteilhafte Identifikation der Bereiche im menschlichen Gehirn, die für eine bestimmte Funktion zuständig sind, gewährleistet. Ebenso ist es Aufgabe der Erfindung, eine Vorrichtung zur Verfügung zu stellen, mit der auf rasche und einfache Weise Areale des menschlichen Gehirns, die für eine bestimmte Funktion zuständig sind, aufgefunden werden können.

Die Erfindung löst diese Aufgabe bei einer Vorrichtung der eingangs genannten Art mit den Merkmalen des Patentanspruchs 1. Dabei ist bei einer Vorrichtung zur Elektrostimulation eines Probanden umfassend eine Anzahl von an das menschliche Gehirn anlegbaren Elektroden zum Auslösen bestimmter elektrischer Reize auf das menschliche Gehirn,

- wobei eine Steuereinheit umfassend eine Stimulationseinheit vorgesehen ist, mit der elektrische Stimuli an die einzelnen oder mehrere Elektroden anlegbar sind,
- wobei die Steuereinheit eine den Elektroden nachgeschaltene Messeinheit zur Bestimmung der an den einzelnen Elektroden anliegenden Spannungen aufweist,
- wobei die Steuereinheit eine Analyseeinheit aufweist, die die einzelnen mit Messelektroden erfassten Messsignale analysiert und im Rahmen einer Vorauswahl aufgrund dieser Analyse einzelne der Elektroden für die Abgabe eines Stimulus auswählt, und
- wobei die Steuereinheit eine, insbesondere vom Menschen betätigbare, Auswahl- und Betätigungseinheit zur Auswahl einer oder mehrerer Elektroden unter den von der Analyseeinheit vorausgewählten Elektroden sowie zur Abgabe eines vorgegebenen elektrischen Stimulus auf die derart ausgewählte(n) Elektrode(n) durch die Stimulationseinheit aufweist, die der Analyseeinheit nachgeschaltet ist und die der Stimulationseinheit vorgeschaltet ist,

vorgesehen dass die Analyseeinheit dazu ausgebildet ist, bei der Vorauswahl die Messsignale an den Messelektroden, insbesondere ausschließlich, auf das Vorliegen von Signalleistungen oder Signalenergien im Bereich von 60 Hz bis 1kHz, insbesondere zwischen 60 Hz und 180 Hz, zu untersuchen.

Ein besonders einfacher Überblick über die Analyseergebnisse wird erreicht, indem die Auswahl- und Betätigungseinheit eine Anzeigeeinheit aufweist, die die Elektroden sowie die von der Analyseeinheit ermittelten Analyseergebnisse, insbesondere die Vorauswahlergebnisse, aufgrund der Analyse für die einzelnen Elektroden an Positionen der Anzeigeeinheit graphische Visualisierungen der Analyseergebnisse darstellt.

Eine einfache Nachprüfung der vorab erzielten Analyseergebnisse ist möglich, indem

- die Auswahl- und Betätigungseinheit im Bereich der Anzeigeeinheit einzelne Auswahl- oder Betätigungselemente aufweist,

- die Auswahl- oder Betätigungselemente jeweils einer Elektrode zugeordnet sind und auf der Anzeigeeinheit im Bereich der Position angeordnet sind, an der die graphischen Visualisierungen für die betreffende Elektrode dargestellt sind, und
- die Auswahl- oder Betätigungselemente zur Auswahl der ihnen zugeordneten Elektrode für die Abgabe eines Stimulus oder zur Abgabe eines Stimulus mit der betreffenden Elektrode ausgebildet sind.

Eine besonders gezielte Stimulation bestimmter Gehirnbereiche kann erreicht werden, indem die Stimulationseinheit einen gleichstromfreien Stimulus auf die Elektroden appliziert.

Um das menschliche Gehirn möglichst wenig zu schädigen, kann vorgesehen sein, dass die Stimulationseinheit den Gleichanteil des Stroms des Stimulus begrenzt, und wobei insbesondere der Stromverlauf des Stimulus einen rechteckigen, dreieckigen oder sinusförmigen Verlauf aufweist.

Um eine automatische Analyse der Gehirnregionen zu erreichen, kann vorgesehen sein, dass eine Erfassungseinheit zur Erfassung der Reaktion des Probanden vorgesehen und an die Steuereinheit angeschlossen ist, wobei die Erfassungseinheit insbesondere durch ein Mikrophon zur Erfassung von Sprache des Probanden oder durch einen Detektor zur Erfassung von Bewegungen des Probanden oder für die Erfassung von elektro-physiologischen Signalen ausgebildet ist.

Um das Ansprechen einzelner Gehirnregionen bei unterschiedlichen Stimulusschwellenwerten zu ermöglichen und eine Überreizung des Gehirns zu vermeiden, kann vorgesehen sein, dass die Stimulationseinheit dazu ausgebildet ist, Stimuli unterschiedlicher, insbesondere ansteigender, Stärke und/oder Dauer auf die einzelnen Elektroden zu applizieren.

Zur manuellen Stimulation kann dabei vorgesehen sein, dass die Stimulationseinheit dazu ausgebildet ist, die einzelnen Stimuli, insbesondere in ansteigender Reihenfolge, bei manueller Betätigung oder automatischer abzugeben.

Zur automatisierten Stimulation kann vorgesehen sein, dass die Steuereinheit die Stimulationseinheit zur Abgabe von Stimuli in ansteigender Reihenfolge ansteuert, bis die Erfassungseinheit eine Reaktion des Probanden feststellt oder die Strombegrenzung erreicht wird.

Um einzelne Funktionen des menschlichen Gehirns vorteilhaft von der allgemeinen Aktivität des menschlichen Gehirns abgrenzen zu können, kann vorgesehen sein, dass die Steuereinheit dazu ausgebildet ist, mittels der Analyseeinheit eine Basismessung durchzuführen, bei der der Proband eine Referenztätigkeit ausführt, und die dabei ermittelten Analyseergebnisse - der jeweiligen messenden Elektrode zugeordnet - als Referenzwerte oder Referenzsignal in einem Referenzspeicher abzuspeichern und zur Verfügung zu halten, und

dass die Analyseeinheit eine Vergleichseinheit aufweist, die die einzelnen ermittelten Analysewerte mit den abgespeicherten Referenzwerten vergleicht, wobei das Analyseergebnis für jede einzelne Elektrode angibt, wie sehr sich die ermittelten Analysewerte von den der jeweiligen Elektrode zugeordneten Referenzwerten unterscheiden.

Besonders vorteilhaft kann zur Aufnahme einzelner Spannungen und einzelnen Punkten des menschlichen Gehirns vorgesehen sein, dass die einzelnen Elektroden in einem Gitter oder mehreren Gittern angeordnet sind, wobei insbesondere die Elektroden innerhalb des jeweiligen Gitters in einer vorgegebenen Struktur angeordnet sind, und/oder dass jede der Elektroden bis auf Randelektroden eine vorgegebene Anzahl von Nachbarelektroden aufweist, die gegenüber der jeweiligen Elektrode an einer vorbestimmten Position angeordnet sind und/oder zueinander gleichen Abstand haben.

Besonders vorteilhaft kann bei den einzelnen Elektroden bzw. bei der Anordnung der Elektroden untereinander vorgesehen sein, dass

a) die einzelnen Elektroden untereinander gleichartig ausgebildet sind, und/oder
b) die einzelnen innerhalb eines Gitters angeordneten Elektroden untereinander gleichartig ausgebildet sind, und/oder
c) dass die Elektroden innerhalb eines Gitters in quadratischer oder sechseckiger Struktur angeordnet sind.

Eine Verbesserung der von den Elektroden ermittelten Spannungen kann erzielt werden, indem jeder der Elektroden ein separater Filter nachgeschaltet ist, der der Analyseeinheit oder der Messeinheit vorgeschaltet ist und der dazu ausgebildet ist

a) bei Vorliegen einer einen vorgegebenen Schwellenwert überschreitenden oder unterschreitenden Signalenergie oder einer von einer Sollform um mehr als einen vorgegebenen Schwellenwert abweichenden Signalform das betreffende Signal zu unterdrücken und nicht an die Analyseeinheit weiterzuleiten, und/oder
b) Signalanteile unterhalb einer Grenzfrequenz von 1Hz bis 5Hz wegzufiltern, und/oder
c) den Mittelwert aller gleichzeitig gemessenen Signalwerte aller Elektroden, insbesondere nur innerhalb desselben Gitters, vom Messwert der betreffenden Elektrode abzuziehen,

d) den, gegebenenfalls gewichteten, Mittelwert aller gleichzeitig gemessenen Signalwerte aller Nachbarelektroden der betreffenden Elektrode, insbesondere nur innerhalb desselben Gitters, vom Messwert der betreffenden Elektrode abzieht, wobei in einem quadratischen Gitter von Elektroden als Nachbarelektroden insbesondere angesehen werden:

> i) die vier unmittelbar an eine Elektrode angrenzenden Elektroden,
> ii) die acht eine Elektrode umgebenden Elektroden, wobei gegebenenfalls die einzelnen Nachbarelektroden mit einem von ihrer Entfernung von der Elektrode abhängigen Gewichtsfaktor gewichtet sind,
> iii) diejenigen vier Elektroden innerhalb eines quadratischen Gitters deren eine Koordinatenposition von der betreffenden Koordinatenposition der Elektrode um zwei abweicht, deren andere Koordinatenposition mit der betreffenden Koordinatenposition der Elektrode übereinstimmt.

Eine besonders bevorzugte Weiterbildung der Erfindung, mit der in Echtzeit Messwerte erstellt werden können, sieht vor,

- dass die Analyseeinheit ausgebildet ist, für jede Elektrode laufend abgeleitete Messwerte zu erstellen, wobei innerhalb eines vorgegebenen Zeitraums erstellte Messwerte zu Fenstern, insbesondere mit einer Länge von 20 ms bis 2 Sekunden, zusammengefasst werden und
- dass die Analyseeinheit ausgebildet ist, insbesondere mittels FFT oder autoregressiver Modelle, wie vorzugsweise LMS, Recursive Least Square oder Kalmann-Filtern von Ordnung 5 bis 50, die Signalenergie des Signals innerhalb des Fensters in einem Frequenzbereich mit einer unteren Frequenz von 60 Hz bis 100 Hz und einer oberen Frequenz in einem Frequenzbereich von 150 Hz bis 1 kHz zu ermitteln, und daraus ein Analysesignal zu erstellen, und gegebenenfalls im Rahmen der Basismessung ein Referenzsignal zu erstellen, und
- dass gegebenenfalls die Analyseeinheit Frequenzbereiche innerhalb des vorgegebenen Fensters, die in einem Bereich um die Netzfrequenz oder ein Vielfaches der Netzfrequenz liegen, nicht für die Bildung der Signalenergie heranzieht.

Zur vorteilhaften Detektion von zusammenhängenden Netzwerken sieht eine bevorzugte Weiterbildung der Erfindung vor, dass die Steuereinheit dazu ausgebildet ist, einen Stimulus im Bereich einer Elektrode, insbesondere mit einem Spannungsstimulus mit einer Frequenz zwischen 1Hz und 100 Hz, abzugeben, und dass die Steuereinheit dazu ausgebildet ist, nach der Abgabe des Stimulus an allen oder einer Anzahl von Elektroden

> a) evozierte Potentiale im abgegebenen Signal der jeweiligen Elektrode zu detektieren, oder
> b) die Bandleistung des abgegebenen Signals der jeweiligen Elektrode, insbesondere im Bereich zwischen 60Hz und kHz zu detektieren, und

dass die Steuereinheit derart alle Elektroden bzw die von den Elektroden erfassten Gehirnregionen darstellt, in denen aufgrund des Stimulus ein evoziertes Potential oder eine erhöhte Bandleistung im Beriech zwischen 60Hz und 1kHz besteht.

Eine besonders bevorzugte Ausführungsform der Erfindung wird anhand der folgenden Zeichnungsfiguren näher dargestellt.

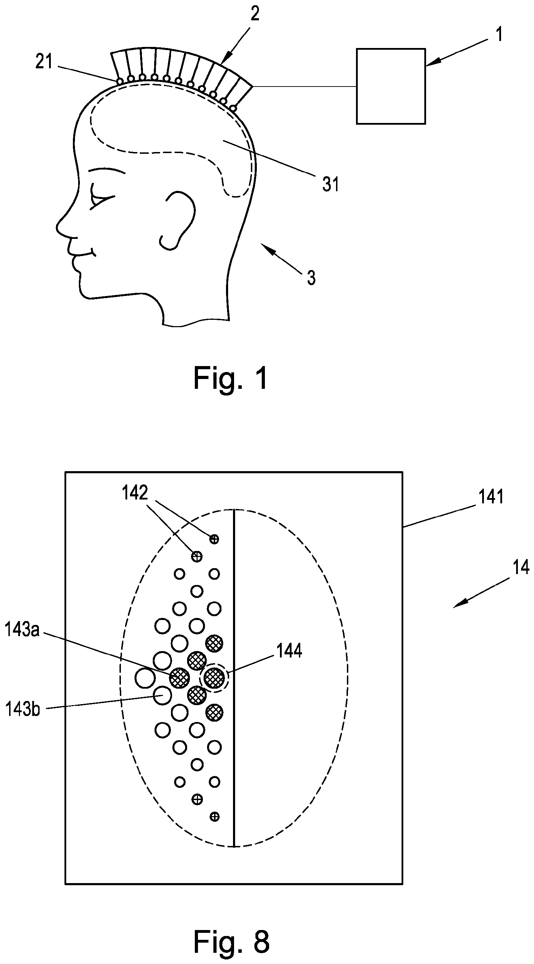

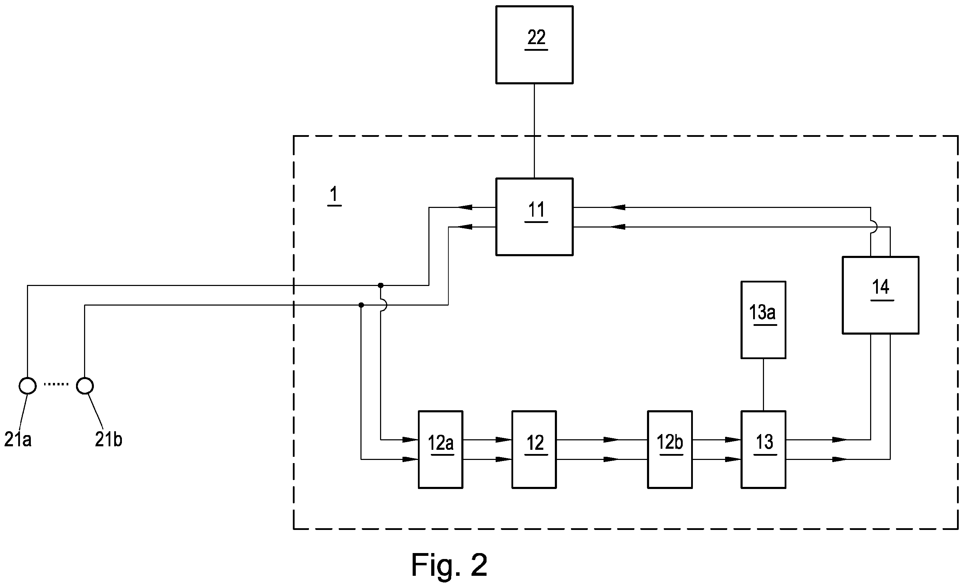

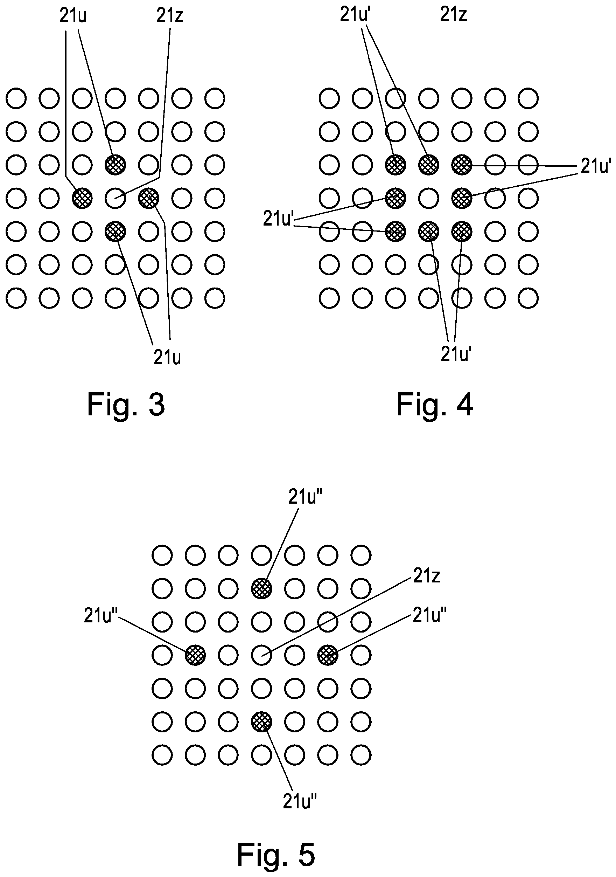

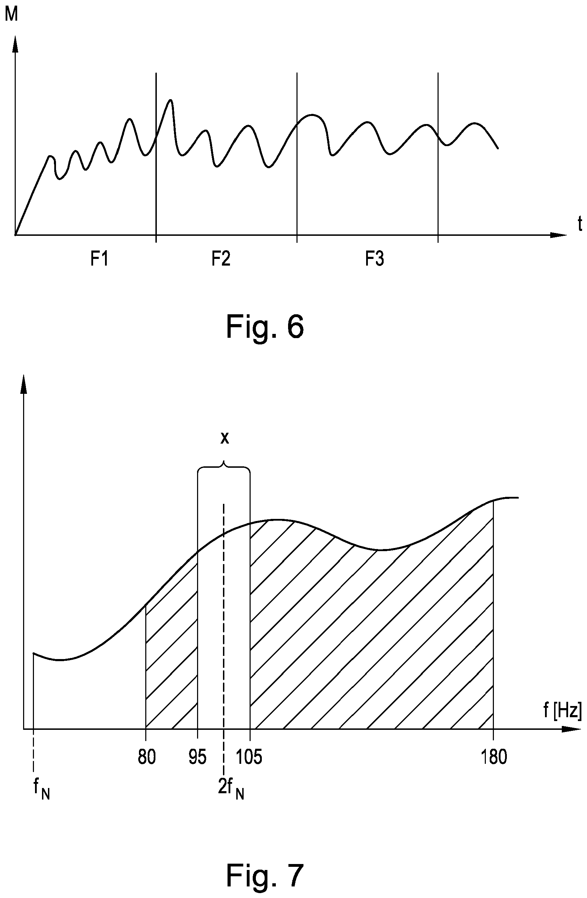

**Fig. 1** zeigt eine Elektrodenanordnung 2 mit einer Anzahl von Elektroden, die an das Gehirn eines Probanden angelegt ist. Die Elektrodenanordnung ist an eine Steuereinheit angeschlossen. **Fig. 2** zeigt eine schematische Darstellung der Steuereinheit der **Fig. 1**. **Fig. 3 bis 5** zeigen unterschiedliche Filter zur Vorverarbeitung der Messsignale. **Fig. 6** zeigt die Erstellung von Fenstern aus den Messsignalen. **Fig. 7** zeigt die Bestimmung der Signalenergie für ein Fenster. **Fig. 8** zeigt schematisch die Anzeige auf einer Anzeigeeinheit.

In **Fig. 1** ist eine Vorrichtung zur Elektrostimulation des Gehirns 31 eines Probanden 3 dargestellt. Diese Vorrichtung umfasst eine Anzahl von an das menschliche Gehirn 31 anlegbaren Elektroden 21, die zu einer Elektrodenanordnung 2 zusammengefasst sind. Diese Elektrodenanordnung 2 ist an eine Steuereinheit 1 angeschlossen.

Grundsätzlich besteht bei den am menschlichen Gehirn anliegenden Elektroden 21 sowohl die Möglichkeit, einzelne Hirnströme über die Elektrode 21 zu messen und die so ermittelten Messsignale M auszuwerten. Andererseits besteht jedoch auch die Möglichkeit, über die Elektroden 21 elektrische Stimuli S an das menschliche Gehirn 31 abzugeben.

Die Elektroden 21 können entweder in einem Gitter oder in mehreren voneinander unabhängigen Gittern angeordnet sein, wobei die einzelnen Elektroden 21 innerhalb des jeweiligen Gitters in einer vorgegebenen Struktur angeordnet sind. Dabei ist vorteilhafterweise vorgesehen, dass jede der Elektroden 21 mit Ausnahme der Randelektroden eine vorgegebene Anzahl von Nachbarelektroden aufweist, wobei die jeweiligen Nachbarelektroden 21 gegenüber der jeweiligen Elektrode 21 in einer vorbestimmten Position angeordnet sind. Innerhalb des Gitters weisen benachbarte Elektroden 21 vorzugsweise zueinander gleichen Abstand auf.

Besonders einfache Ausbildungen von Gittern können dadurch erzielt werden, dass die einzelnen Elektroden 21 untereinander gleichartig ausgebildet sind bzw. dass die einzelnen innerhalb eines Gitters angeordneten Elektroden 21 untereinander gleichartig ausgebildet sind. Innerhalb eines Gitters können die Elektroden 21 in quadratischer oder sechseckiger oder sonst regelmäßiger Struktur angeordnet sein.

Um die Geometrie der einzelnen Gitter richtig abbilden zu können, können unterschiedliche Verarbeitungsprogramme gewählt werden, die eine geometrische Abbildung des Elektrodengitters sowie eine lagerichtige Darstellung der einzelnen Elektroden ermöglichen.

Die in **Fig. 2** näher dargestellte Steuereinheit 1 umfasst eine Stimulationseinheit 11, die dazu in der Lage ist, elektrische Stimuli S an die einzelnen Elektroden 21 des menschlichen Gehirns 31 abzugeben. Darüber hinaus umfasst die Steuereinheit 1 auch eine den Elektroden 21 nachgeschaltete Messeinheit 12. Mit dieser Messeinheit 12 können einzelne an den Elektroden 21 anliegende Messsignale M in Form von Spannungen ermittelt und weiter verarbeitet werden. Die so ermittelten bzw. von der Messeinheit 12 gemessenen Messsignale M sind einer Analyseeinheit 13 zugeführt, die die einzelnen an den Messelektroden 21 anliegenden Messsignale M analysiert und aufgrund dieser Analyse eine Vorauswahl durchführt. Bei dieser Analyse werden einzelne Elektroden 21, bei denen aufgrund der Analyse besondere Eigenschaften in den Signalen festgestellt wurden, für die Abgabe eines Stimulus S ausgewählt. Von Vorteil kann auch sein, umliegende Elektroden 21 mit einem Simulus S zu beaufschlagen, um die Gehirnregion genauer untersuchen zu können. Eine solche Auswahl erfolgt vorzugsweise dadurch, dass die an den Elektroden 21 anliegenden Messsignale M darauf analysiert werden, ob in einem bestimmten Frequenzbereich zwischen 60 Hz und 1 kHz, insbesondere zwischen 60 Hz und 170 Hz erhöhte Signalenergien vorhanden sind.

Der Messeinheit 12 kann für jede der Elektroden 21 jeweils ein separater Filter 12a, 12b nachgeschaltet sein. Dieser Filter 12a kann entweder im Signalweg vor der Messeinheit oder im Signalweg zwischen der Messeinheit 12 und der Analyseeinheit 13 angeordnet sein. Ist der Filter 12a vor der Messeinheit 12 angeordnet, kann der Filter 12a vorzugsweise als analoger Filter 12a ausgebildet sein. Im Signalweg zwischen der Messeinheit 12 und der Analyseeinheit 13 kann der Filter 12b vorzugsweise als digitaler Filter 12b ausgebildet sein.

Eine mögliche Ausführungsform eines Filters 12a, 12b unterdrückt das betreffende Signal bei Vorliegen einer Signalenergie im Messsignal M, die einen vorgegebenen Schwellenwert überschreitet oder unterschreitet oder bei einer Signalform des Messsignals M, die von einer vorgegebenen Sollform um mehr als einen vorgegebenen Schwellenwert abweicht. Dieses Signal wird in diesem Fall nicht an die Analyseeinheit 13, gegebenenfalls auch nicht an die Messeinheit 12 weitergeleitet.

Zusätzlich oder alternativ kann auch ein Filter 12a, 12b im Signalweg vor der Messeinheit 12 oder zwischen der Messeinheit 12 und der Analyseeinheit 13 angeordnet sein, der Signalanteile unterhalb einer vorgegebenen Grenzfrequenz wegfiltert. Diese Grenzfrequenz kann zwischen 0.1 Hz und 5 Hz gewählt werden.

Eine weitere Möglichkeit der Funktionsweise eines zusätzlichen oder alternativen Filters 12a, 12b besteht darin, den Mittelwert aller gleichzeitig gemessenen Signalwerte aller Elektroden 21 vom Messwert der betreffenden Elektrode 21 abzuziehen. Dies ermöglicht eine Unterdrückung von Einflüssen, die Spannungsschwankungen auf sämtlichen Elektroden 21 verursachen. Sofern, wie dies im nachfolgenden Ausführungsbeispiel dargestellt wird, mehrere Gitter von Elektroden 21 verwendet werden, kann ein Filter 12a, 12b dazu ausgebildet sein, den Mittelwert aller gleichzeitig gemessenen Signalwerte der Elektroden 21 nur innerhalb desselben Gitters von den einzelnen Messwerten der betreffenden Elektroden 21 abzuziehen.

Darüber hinaus kann die Nachbarschaft einzelner Nachbarelektroden 21u innerhalb der Elektrodenanordnung 2 bzw. innerhalb eines Elektrodengitters ausgenutzt werden, um Effekte in der Umgebung um eine Elektrode 21z auszublenden. Dabei besteht die Möglichkeit, einen Mittelwert aller gleichzeitig gemessenen Signalwerte aller Nachbarelektroden 21u einer betreffenden Elektrode 21 vom Messwert der Elektrode 21 abzuziehen, um auf diese Weise einen Filterwert zu ermitteln. Sofern das Elektrodengitter als quadratisches Elektrodengitter ausgebildet ist, das heißt die Elektroden 21 innerhalb eines Elektrodengitters jeweils eine rechte, eine linke, eine obere und eine untere Nachbarelektrode 21u enthalten, können bevorzugt folgende Filtermaßnahmen unter Verwendung von Nachbarelektroden durchgeführt werden.

Der Mittelwert kann ermittelt werden durch Mittelung der unmittelbar an die jeweilige Elektrode 21z angrenzenden Elektroden 21u (**Fig. 3**). In diesem Fall wird der Filterwert errechnet, indem vom gemessenen Signalwert die Summe der gemessenen Signalwerte der Nachbarelektroden geteilt durch 4 abgezogen wird. Der so ermittelte Wert entspricht im Wesentlichen dem diskret ermittelten Laplace-Operator bzw. einem Vielfachen des diskret ermittelten Laplace-Operators.

Alternativ besteht auch die Möglichkeit, in einem quadratischen Elektrodengitter die acht eine Elektrode 21z umgebenden Nachbarelektroden 21u' für die Ermittlung des Mittelwerts heranzuziehen (**Fig. 4**). Dabei können diejenigen Nachbarelektroden 21u', die gegenüber der zentralen Elektrode 21z diagonal liegen, mit einem geringerem Gewichtsfaktor gewichtet werden. Insbesondere kann dieser Gewichtsfaktor vom Abstand der Nachbarelektroden abhängen, sodass diagonal liegende Nachbarelektroden 21u' mit einem Faktor 1 durch $\sqrt{2}$ schwächer gewichtet sind als unmittelbar anliegende Nachbarelektroden 21u'.

Darüber hinaus besteht auch die Möglichkeit, anstelle der vier unmittelbar an die Elektrode 21z angrenzenden Elektroden 21u diejenigen vier Elektroden 21u" innerhalb eines quadratischen Gitters für die Bestimmung des Mittelwerts heranzuziehen, deren eine Koordinatenposition von der betreffenden Koordinatenposition der Elektrode 21z um zwei abweicht, deren andere Koordinatenpositionen mit der betreffenden Koordinatenposition der Mittelelektrode übereinstimmt (**Fig. 5**).

Eine bevorzugte Funktionsweise der Analyseeinheit 13 wird im Folgenden näher dargestellt:
Die Analyseeinheit 13 ist dazu ausgebildet, für jede einzelne Elektrode 21 laufend abgeleitete und allenfalls gefilterte Messwerte zu verarbeiten, wobei innerhalb eines vorgegebenen Zeitraums erstellte Messwerte zu Fenstern zusammengefasst werden (**Fig. 6**). In einer bevorzugten Ausführungsform der Erfindung weisen diese Fenster $F_1$, $F_2$, $F_3$ eine Länge von 200 ms auf. Grundsätzlich ist es jedoch ohne weiteres möglich, auch Fenster mit einer Länge von 20 ms bis zu 15 Sekunden zu erstellen. Innerhalb dieser Fenster werden die Messwerte mit einer Abtastfrequenz von 1000-5000 Hz abgetastet.

Die Analyseeinheit 13 ist dazu ausgebildet, die Signalenergie des Signals innerhalb des Fensters in einem Frequenzbereich mit einer unteren Frequenz von zwischen 60 und 100 Hz und einer oberen Frequenz von zwischen 150 Hz bis 1 kHz zu ermitteln. Um jedes Fenster $F_1$, $F_2$, $F_3$ mit einer vorgegebenen Dauer wird jeweils eine Signalenergie angegeben. Die Berechnung der Signalenergie kann beispielsweise mittels FFT oder mittels autoregressiver Modelle, wie beispielsweise LMS, Recursive Least Square oder Kalmann-Filtern von Ordnung zwischen 5 und 100 ermittelt werden.

Für jedes Fenster $F_1$, $F_2$, $F_3$ steht jeweils ein Analysewert zur Verfügung, der die Signalenergie im betreffenden Fenster $F_1$, $F_2$, $F_3$ angibt. Die einzelnen Analysewerte werden zu einem Analysesignal A zusammengefasst, das für jedes Fenster jeweils einen Analysewert in Form der Signalenergie aufweist.

Bei einer bevorzugten Ausführungsform der Erfindung werden Frequenzbereiche innerhalb eines vorgegebenen Frequenzfensters, die in einem Bereich um die Netzfrequenz oder ein Vielfaches der Netzfrequenz liegen, nicht für die Bildung der Signalenergie herangezogen. Dies kann beispielsweise bei einer Netzfrequenz von 50 Hz bevorzugt im Bereich des Doppelten der Netzfrequenz, das heißt im Bereich von 100 Hz erfolgen, wobei bei der Bestimmung der Signalenergie Energien im Bereich von zum Beispiel zwischen 95 Hz und 105 Hz ausgefiltert werden (**Fig. 7**).

Eine besonders bevorzugte Art der Analyse der einlangenden Messwerte kann vorgenommen werden, indem mittels eine Basismessung durchgeführt wird, bei der der Proband 3 eine geistige Referenztätigkeit ausführt, beispielsweise entspannt oder an nichts denkt. Mittels der einzelnen Elektroden 21 werden aus den Messsignalen M wie vorstehend dargestellt Analysewerte abgeleitet, wobei diese Werte den einzelnen Elektroden 21 zugeordnet werden und der Analyse der Analyseeinheit 13 unterzogen werden. Die aus den Messsignalen M abgeleiteten Analysewerte werden in einem Referenzspeicher 13a abgespeichert und in diesem zur Verfügung gehalten. Der Referenzspeicher 13a ist an die Analyseeinheit 13 angeschlossen. Die Analyseeinheit 13 verfügt darüber hinaus über eine Vergleichseinheit, die die an den Elektroden 21 ermittelten Spannungen bei konkreten geistigen Tätigkeiten ermittelt und daraus Analysewerte ableitet. Diese Werte werden mit den im Referenzspeicher 13a abgespeicherten Referenzwerten verglichen. Aufgrund des Vergleichs wird eine Analyseergebnis ermittelt, das für jede einzelne Elektrode 21 angibt, wie sehr sich die ermittelten Messsignale von den der jeweiligen Elektrode 21 zugeordneten Referenzwerten unterscheiden.

Besonders bevorzugt kann die Analyseeinheit 13 derart ausgebildet sein, dass sie aus dem bei der Basismessung erzeugten Signal die Signalenergie für die einzelnen Zeitfenster, wie in **Fig. 8** dargestellt, ableitet und daraus ein Referenzsignal R erzeugt, das sie im Referenzspeicher 13a abspeichert. Im Zuge dieser Basismessung wird für jede einzelne Elektrode 21 ein separates Referenzsignal R abgespeichert, das angibt, welche elektrischen Signale vom Gehirn des Probanden 3 bei einer Referenztätigkeit abgegeben werden.

Die Analyseeinheit 13 ist ferner dazu ausgebildet, Koeffizienten k zu ermitteln, die für jede Elektrode 21 angeben, ob sich das vom Referenzsignal R abgeleitete Signal für die betreffende Elektrode 21 und das vom momentan erfassten Messsignal M abgeleitete Analysesignal A voneinander unterscheiden. Die Analyseeinheit 11 hält für eine Anzahl von Elektroden 21, insbesondere für alle Elektroden 21, einen solchen Koeffizienten k zur Verfügung. Besonders bevorzugt ist es möglich, dass die Analyseeinheit 13 die vorliegenden Koeffizienten k derart normiert, dass alle Koeffizienten k durch denselben maximalen Koeffizienten $k_{max}$ dividiert werden. Alternativ besteht auch die Möglichkeit, die einzelnen Koeffizienten k derart mit demselben Gewichtswert zu gewichten, dass die Summe aller Koeffizienten k aller Elektroden 21 einen vorgegebenen Wert, beispielsweise 1, hat.

Eine besonders bevorzugte Variante zur Bestimmung, ob sich ein Referenzsignal R von einem im Zuge einer weiteren Analyse ermittelten Analysesignal A unterscheidet, wird im Folgenden näher dargestellt. Hierfür werden jeweils diejenigen Werte für die Signalenergie herangezogen, die von der Analyseeinheit 13 im Rahmen der Basismessung für das Referenzsignal R ermittelt wurden einerseits und diejenigen Werte, die im Zuge der Analyse des jeweils aktuellen Messsignals S ermittelt wurden, wobei das so ermittelte Analysesignal A ebenfalls wiederum einzelne Werte für die Signalenergie für einzelne Zeitfenster $F_1$, $F_2$, $F_3$ zur Verfügung stehen. Es liegt also eine Anzahl von Signalenergien vor, die im Zuge der Basismessung ermittelt wurden sowie eine Anzahl von Signalenergien, die im Zuge der aktuellen Messung ermittelt wurden. In einem ersten Schritt werden Zahlenpaare erstellt, deren erster Wert die jeweilige Signalenergie im Zuge der Messung bzw. Basismessung ist und deren zweiter Wert angibt, ob die jeweilige Signalenergie aus der Messung oder aus der Basismessung stammt. Beispielsweise kann für den Fall, dass die Signalenergie aus der Basismessung stammt, der Wert -1 und für den Fall, dass die Signalenergie aus der Messung stammt, der Wert +1 vergeben werden. Die jeweils verwendeten Werte sind für die weiteren Berechnungen nicht erheblich, solange diese sich numerisch gut voneinander unterscheiden lassen.

Ob sich die Signale der Basismessung von den Signalen der aktuellen Messung gut unterscheiden lassen, kann durch den quadrierten Korrelationskoeffizienten $r^2$ einfach angegeben werden.

$$r^2 = \frac{\mathrm{cov}(x,y)^2}{\mathrm{var}(x)\mathrm{var}(y)}$$

Die Anzahl der im Zuge der Basismessung ermittelten Signalenergien wird mit $n_1$ bezeichnet, die Anzahl der im Zuge der aktuellen Messung ermittelten Signalenergien wird mit $n_2$ bezeichnet. Eine einfachere Möglichkeit, numerisch effizient den Korrelationskoeffizienten zu bestimmen, liegt darin, die Summe der einzelnen Signalenergien $X_i$ sowie die Summe der Quadrate der einzelnen Signalenergien getrennt danach, ob sie im Zuge der Basismessung oder im Zuge der aktuellen Messung ermittelt worden sind, einzeln abzuspeichern und vorrätig zu halten.

$$s_k := \sum_i x_i^{(k)}, \quad q_k := \sum_i x_i^{(k)^2}$$

Die für die Berechnung des Korrelationskoeffizienten k erforderlichen Werte der Kovarianz cov (x, y) sowie der Varianz var (x) können aus den vorliegenden Summen und Quadratsummen wie folgt ermittelt werden:

$$\mathrm{cov}(x,y) = \frac{s_1 - s_2}{n_1 + n_2} - \frac{(s_1 + s_2)(n_1 - n_2)}{(n_1 + n_2)^2} = 2\frac{s_1 n_2 - s_2 n_1}{(n_1 + n_2)^2}$$

$$\mathrm{var}(x) = \frac{q_1 + q_2}{n_1 + n_2} - \frac{(s_1 + s_2)^2}{(n_1 + n_2)^2}$$

$$\mathrm{var}(y) = 1 - \frac{(n_1 - n_2)^2}{(n_1 + n_2)^2} = \frac{4n_1 n_2}{(n_1 + n_2)^2}$$

Dadurch ergibt sich der Korrelationskoeffizient wie folgt:

$$r^2 = \frac{1}{n_1 n_2} \frac{(s_1 n_2 - s_2 n_1)^2}{(n_1 + n_2)(q_1 + q_2) - (s_1 + s_2)^2} = \frac{s_1^2/n_1 + s_2^2/n_2 - G}{q_1 + q_2 - G} \quad ,$$

wobei ein Faktor G eingeführt werden kann, der zu einer numerischen Vereinfachung der Berechnung führt:

$$G := \frac{(s_1 + s_2)^2}{n_1 + n_2}$$

Die vorliegende Vorrichtung weist eine Auswahl- und Betätigungseinheit 14 auf, mit der eine oder mehrere Elektroden 21 unter den von der Analyseeinheit vorausgewählten Elektroden zur Abgabe eines vorgegebenen elektrischen Stimulus S ausgewählt werden können. Die Auswahl- und Betätigungseinheit 14 ist der Analyseeinheit 13 nachgeschaltet und der Stimulationseinheit 11 vorgeschaltet. Im vorliegenden bevorzugten Ausführungsbeispiel einer Auswahl- und Betätigungseinheit 14, das in **Fig. 8** dargestellt ist, weist die Auswahl- und Betätigungseinheit 14 eine Anzeigeeinheit 141 auf, die die Elektroden 21 sowie die von der Analyseeinheit 13 ermittelten Analyseergebnisse, insbesondere im vorliegenden Fall die aufgrund des Korrelationskoeffizienten k, vorzugsweise durch Schwellenwertvergleich ermittelten Vorauswahlergebnisse, aufgrund der Analyse für die einzelnen Elektroden 21 an Positionen 142 der Anzeigeeinheit in Form von graphischen Visualisierungen 143a, 143b darstellt. Die dargestellte Auswahl- und Betätigungseinheit 14 weist im Bereich der Anzeigeeinheit 141 einzelne Auswahl- und Betätigungselemente 144 für jede einzelne Elektrode 21 auf. Die Auswahl- und Betätigungselemente 144 sind jeweils einer Elektrode 21 zugeordnet und auf der Anzeigeeinheit 141 im Bereich der Position 142 angeordnet, an der auch die graphischen Visualisierungen 143a, 143b für die betreffende Elektrode 21 dargestellt sind. Die Auswahl- und Betätigungselemente 144 zur Auswahl der ihnen zugeordneten Elektrode 21 für die Abgabe eines Stimulus S oder zur Abgabe eines Stimulus S mit der betreffenden Elektrode 21 ausgebildet. Eine besonders bevorzugte Auswahl von Elektroden 21 durch die Auswahl und Betätigungseinheit 144 erfolgt, indem bei Auswahl einer Elektrode 21 jeweils eine benachbarte Elektrode 21 mit ausgewählt wird. Ein Stimulus S wird in Form eines Stroms zwischen den beiden so ausgewählten Elektroden 21 abgegeben. Ist von der Auswahl- und Betätigungseinheit 14 aufgrund der Analyse eine Elektrode 21 vorausgewählt, so schlägt die Auswahl- und Betätigungseinheit 14 eine zu dieser ausgewählten Elektrode 21 benachbarte Elektrode 21 zur Auswahl vor oder wählt diese von selbst aus. Bei Betätigung wird ein Stimulus S in Form eines Stroms zwischen diesen beiden Elektroden 21 abgegeben. Von Vorteil ist auch, dass mehrere Elektroden 21 gleichzeitig oder rasch hintereinander stimuliert werden können, um den Effekt zu verstärken und das Mapping schneller zu machen.

Der abgegebene elektrische Stimulus S weist bevorzugterweise einen Gleichstromanteil auf, der unterhalb eines vorgegebenen Schwellenwerts liegt. Dies kann entweder dadurch erfolgen, dass die Stimulationseinheit 11 einen gleichstromfreien Stimulus S auf die Elektroden 21 appliziert. Alternativ kann der Gleichstromanteil auch dadurch reduziert werden, indem der Stimulus 21 zeitlich begrenzt ist. In beiden Fällen können rechteckige Impulse zur Stimulation verwendet werden, beispielsweise mit einer Dauer von 1 ms und einer Stromstärke von 10 mA.

Besonders vorteilhaft kann zur Stimulation mit unterschiedlichen Stimulationsschwellenwerten vorgesehen sein, dass die Stimulationseinheit 11 dazu ausgebildet ist, Stimuli S unterschiedlicher und ansteigender Stärke auf die einzelnen Elektroden 21 zu applizieren. Die Stimulationseinheit 11 kann auch ausgebildet sein, die einzelnen Stimuli S bei manueller Betätigung abzugeben.

Alternativ besteht jedoch auch die Möglichkeit, dass Stimulationseinheit 11 zur Abgabe Stimuli in ansteigender Reihenfolge angesteuert wird, bis eine Erfassungseinheit 22 eine Reaktion des Probanden 3 feststellt bzw. erfasst. Die Erfassungseinheit 22 ist an die Steuereinheit angeschlossen und kann beispielsweise durch ein Mikrofon zur Erfassung von Sprache des Probanden 3 oder durch einen Detektor zur Erfassung von Bewegungen des Probanden 3 ausgebildet sein. Die Erfassungseinheit 22 kann auch durch eine manuelle Betätigung ersetzt werden, indem der Arzt die Reaktion des Probanden erfasst und die Stimulation entsprechend beendet.

Zusätzlich besteht noch die Möglichkeit, Entladungen nach der Stimulation (after discharges) zu detektieren. Diese werden durch die Elektrostimulation im Gehirn ausgelöst und zeigen an, dass allenfalls ein epileptischer Anfall droht. In einem solchen Fall kann vorgesehen sein, dass der für die Elektrostimulation herangezogene Strom nicht weiter erhöht wird bzw. die Stimulation beendet wird. Durch eine Warnung wird der Arzt auf die After-discharges hingewiesen.

Sofern eine solche Entladung (after discharge) erkannt wird, besteht die Möglichkeit, weitere Stimuli abzugeben, um manuell einen epileptischen Anfall zu unterdrücken.

In einer weiteren vorteilhaften Ausführungsform der Erfindung besteht die Möglichkeit, die einzelnen im Zuge der Analyse der Messsignale ermittelten Zwischenergebnisse oder die einzelnen Messdaten unmittelbar anzuzeigen.

Weiters besteht die Möglichkeit, eine Spektralinformation der ermittelten Rohmessdaten anzuzeigen, insbesondere um Störungen vorzeitig erkennen zu können. Darüber hinaus besteht die Möglichkeit, dass einzelne Elektroden, die nur schlechten Kontakt haben oder insgesamt schadhaft sind, von der Messung ausgeschlossen werden. Für solche Elektroden werden dann insgesamt keine Messdaten erhoben und es wird auch für solche Elektroden keine Analyse durchgeführt.

In einer weiteren bevorzugten Ausführungsform der Erfindung besteht die Möglichkeit, dass die Signalmasse beliebig ausgewählt bzw. auf einen beliebigen Spannungswert gelegt werden kann. Dies ist insbesondere vorteilhaft um zu vermeiden, dass eine Messung unmöglich wird, wenn die während der Messung verwendete Masse störungsbehaftet ist.

Grundsätzlich besteht die Möglichkeit, dass eine Vielzahl unterschiedlicher geistiger Tätigkeiten dem Probanden vorgegeben werden können, beispielsweise die Lösung eines Rubic Cubes, Hörübungen, das Benennen von Bildern, Kussbewegungen, Zungenbewegungen, Lesen, Rechnen, Erinnerungen etc. Hierbei besteht auch die Möglichkeit, dass unterschiedliche Tätigkeiten wiederholt werden können, um insgesamt eine bessere Qualität der Aufnahmen zu erlangen. Darüber hinaus besteht auch die Möglichkeit, dass die Qualität einzelner Aufnahmen angezeigt wird. Die Anzahl der geistigen Tätigkeiten, die einem Menschen vorgegeben werden, braucht nicht notwendigerweise beschränkt zu sein. Es besteht auch die Möglichkeit, dass vom Probanden oder vom Versuchsleiter zusätzliche gedankliche Tätigkeiten vorgegeben werden.

Weitere Möglichkeiten, Messsignale bei unterschiedlichen Zuständen des menschlichen Gehirns zu erstellen, bestehen darin, den Körper taktil, auditorisch oder visuell zu stimulieren.

Darüber hinaus ist es auch möglich, einzelne Analyseergebnisse sowie die Gesamtheit aller Analyseergebnisse am Ende der Messung auszudrucken und abzuspeichern.

Darüber hinaus besteht die Möglichkeit, in den einzelnen abgespeicherten Ergebnissen zu vermerken, zwischen welchen Elektroden bzw. an welchen Elektroden automatische oder manuelle Stimulationen vorgenommen werden. Solche Stimulationen können in den einzelnen abgespeicherten oder ausgedruckten Messergebnissen vermerkt werden.

Eine weiter Ausführungsform erlaubt, kortikale Netzwerke zu erkennen, indem eine bekannte Gehirnregion mittels zweier Elektroden (21) stimuliert wird, vorzugsweise mit 1-50 Hz. Bei allen anderen Elektroden werden evozierte Potentiale berechnet und gegebenenfalls visualisiert werden. Diese können durch ereignisbezogene Mittelung mit Trend- und Baselinekorrektur ermittelt werden. Der Vorteil liegt darin, dass mit dieser Vorgehensweise nur eine einzige Region, beispielsweise die Brocas Area stimuliert werden braucht, um das gesamte Sprachnetzwerk des Gehirns zu erkennen. Als Grundlage dient dazu das high-gamma-Mapping, um eine bestimmte Gehirnregion zu erkennen, die anschließend elektrisch stimuliert wird, um das Netzwerk zu erkennen.

Eine weiter Besonderheit liegt dass neben dem evozierten Potential oder anstelle des evozierten Potentials auch die Bandleistung im Bereich von 60-1000 Hz berechnet werden kann, um auf diese Weise kortikale Netzwerke, wie beispielsweise das Sprachnetzwerk, durch High-Gamma Analyse zu detektieren.

## Patentansprüche

1. Vorrichtung zur Elektrostimulation eines Probanden (3) umfassend eine Anzahl von an das menschliche Gehirn (31) anlegbaren Elektroden (21) zum Auslösen bestimmter elektrischer Reize auf das menschliche Gehirn (31),

   - wobei eine Steuereinheit (1) umfassend eine Stimulationseinheit (11) vorgesehen ist, mit der elektrische Stimuli (S) an die einzelnen oder mehrere Elektroden (21) anlegbar sind,
   - wobei die Steuereinheit (1) eine den Elektroden (21) nachgeschaltete Messeinheit (12) zur Bestimmung der, gegebenenfalls nach Vorgabe von unterschiedlichen geistigen Tätigkeiten an den Probanden, an den einzelnen Elektroden (21) anliegenden Spannungen aufweist,
   - wobei die Steuereinheit (1) eine Analyseeinheit (13) aufweist, die die einzelnen mit Messelektroden (21) erfassten Messsignale analysiert,
   - wobei die Analyseeinheit (13) dazu ausgebildet ist, im Rahmen einer Vorauswahl aufgrund der Analyse der erfassten Messsignale einzelne der Elektroden (21) für die Abgabe eines Stimulus auszuwählen,
   - wobei die Analyseeinheit (13) dazu ausgebildet ist, bei der Vorauswahl die Messsignale (M) an den Messelektroden, insbesondere ausschließlich, auf das Vorliegen von Signalleistungen oder Signalenergien im Bereich von 60 Hz bis 1kHz, insbesondere zwischen 60 Hz und 180 Hz, zu untersuchen und
   - wobei die Steuereinheit (1) eine, insbesondere vom Menschen betätigbare, Auswahl- und Betätigungseinheit (14) zur Auswahl einer oder mehrerer Elektroden (21) unter den von der Analyseeinheit (13) vorausgewählten Elektroden (21) sowie zur Abgabe eines vorgegebenen elektrischen Stimulus (S) auf die derart ausgewählte(n) Elektrode(n) (21) durch die Stimulationseinheit (11) aufweist, die der Analyseeinheit (13) nachgeschaltet ist und die der Stimulationseinheit (11) vorgeschaltet ist,
   **dadurch gekennzeichnet,**
   - **dass** die Steuereinheit dazu ausgebildet ist, mittels der Analyseeinheit (13) eine Basismessung durchzuführen, bei der der Proband (3) eine Referenztätigkeit ausführt, und die dabei ermittelten Analyseergebnisse - der jeweiligen messenden Elektrode (21) zugeordnet - als Referenzwerte oder Referenzsignal in einem Referenzspeicher (13a) abzuspeichern und zur Verfügung zu halten, und
   - **dass** die Analyseeinheit (13) eine Vergleichseinheit aufweist, die die einzelnen ermittelten Analysewerte mit den abgespeicherten Referenzwerten vergleicht, wobei das Analyseergebnis für jede einzelne Elektrode (21) angibt, wie sehr sich die ermittelten Analysewerte von den der jeweiligen Elektrode (21) zugeordneten Referenzwerten unterscheiden.

2. Vorrichtung nach Anspruch 1, **dadurch gekennzeichnet, dass** die Auswahl- und Betätigungseinheit (14) eine Anzeigeeinheit (141) aufweist, die die Elektroden (21) sowie die von der Analyseeinheit (13) ermittelten Analyseergebnisse, insbesondere die Vorauswahlergebnisse, aufgrund der Analyse für die einzelnen Elektroden (21) an Positionen (142) der Anzeigeeinheit (141) graphische Visualisierungen der Analyseergebnisse darstellt (143a, 143b).

3. Vorrichtung nach Anspruch 2, **dadurch gekennzeichnet,**

- **dass** die Auswahl- und Betätigungseinheit (14) im Bereich der Anzeigeeinheit (141) einzelne Auswahl- oder Betätigungselemente (144) aufweist,
- **dass** die Auswahl- oder Betätigungselemente (144) jeweils einer Elektrode (21) zugeordnet sind und auf der Anzeigeeinheit (141) im Bereich der Position (142) angeordnet sind, an der die graphischen Visualisierungen (143a, 143b) für die betreffende Elektrode (21) dargestellt sind, und
- **dass** die Auswahl- oder Betätigungselemente (144) zur Auswahl der ihnen zugeordneten Elektrode (21) für die Abgabe eines Stimulus (S) oder zur Abgabe eines Stimulus (S) mit der betreffenden Elektrode (21) ausgebildet sind.

4. Vorrichtung nach einem der vorangehenden Ansprüche, **dadurch gekennzeichnet, dass** die Auswahl- und Betätigungseinheit (14) die Auswahl einer von der Analyseeinheit (13) vorausgewählten Elektroden (21) sowie einer zu dieser benachbarten Elektroden (21) ermöglicht, und die Stimulationseinheit (11) dazu ausgebildet ist, einen Stimulus (S) in Form eines Stroms zwischen den beiden Elektroden (21) abzugeben, wobei insbesondere der Gleichstromanteil des zwischen den benachbarten Elektroden (21) als Stimulus (S) fließenden Stroms unterhalb eines vorgegebenen Schwellenwerts liegt, vorzugsweise

a) indem die Stimulationseinheit (11) einen gleichstromfreien Stimulus (S) auf die Elektroden (21) appliziert, oder
b) indem die Stimulationseinheit (11) den Gleichanteil des Stroms des Stimulus (S) begrenzt, und wobei insbesondere der Stromverlauf des Stimulus einen rechteckigen, dreieckigen oder sinusförmigen Verlauf aufweist.

5. Vorrichtung nach einem der vorangehenden Ansprüche, **dadurch gekennzeichnet, dass** eine Erfassungseinheit (22) zur Erfassung der Reaktion des Probanden (3) vorgesehen und an die Steuereinheit (1) angeschlossen ist, wobei die Erfassungseinheit (22) insbesondere durch ein Mikrophon zur Erfassung von Sprache des Probanden (3) oder durch einen Detektor zur Erfassung von Bewegungen des Probanden (3) oder für die Erfassung von elektrophysiologischen Signalen ausgebildet ist.

6. Vorrichtung nach einem der vorangehenden Ansprüche, **dadurch gekennzeichnet, dass** die Stimulationseinheit (11) dazu ausgebildet ist, Stimuli (S) unterschiedlicher, insbesondere ansteigender, Stärke und/oder Dauer auf die einzelnen Elektroden (21) zu applizieren, wobei insbesondere,

a) die Stimulationseinheit (11) dazu ausgebildet ist, die einzelnen Stimuli (S), insbesondere in ansteigender Reihenfolge, bei manueller Betätigung oder automatischer abzugeben, oder
b) die Steuereinheit (1) die Stimulationseinheit (11) zur Abgabe von Stimuli (S) in ansteigender Reihenfolge ansteuert, bis die Erfassungseinheit (22) eine Reaktion des Probanden (3) feststellt oder die Strombegrenzung erreicht wird

7. Vorrichtung nach einem der vorangehenden Ansprüche, **dadurch gekennzeichnet, dass** die einzelnen Elektroden (21) in einem Gitter oder mehreren Gittern angeordnet sind, wobei insbesondere die Elektroden (21) innerhalb des jeweiligen Gitters in einer vorgegebenen Struktur angeordnet sind, und/oder dass jede der Elektroden (21) bis auf Randelektroden eine vorgegebene Anzahl von Nachbarelektroden (21) aufweist, die gegenüber der jeweiligen Elektrode an einer vorbestimmten Position angeordnet sind und/oder zueinander gleichen Abstand haben.

8. Vorrichtung nach einem der vorangehenden Ansprüche, **dadurch gekennzeichnet, dass**

a) die einzelnen Elektroden (21) untereinander gleichartig ausgebildet sind, und/oder
b) die einzelnen innerhalb eines Gitters angeordneten Elektroden (21) untereinander gleichartig ausgebildet sind, und/oder
c) dass die Elektroden (21) innerhalb eines Gitters in quadratischer oder sechseckiger Struktur angeordnet sind.

9. Vorrichtung nach einem der vorangehenden Ansprüche, **dadurch gekennzeichnet, dass** jeder der Elektroden (21) ein separater Filter (12a, 12b) nachgeschaltet ist, der der Analyseeinheit (13) oder der Messeinheit (12) vorgeschaltet ist und der dazu ausgebildet ist

a) bei Vorliegen einer einen vorgegebenen Schwellenwert überschreitenden oder unterschreitenden Signalenergie oder einer von einer Sollform um mehr als einen vorgegebenen Schwellenwert abweichenden Signalform das betreffende Signal zu unterdrücken und nicht an die Analyseeinheit weiterzuleiten, und/oder

b) Signalanteile unterhalb einer Grenzfrequenz von 1Hz bis 5Hz wegzufiltern, und/oder

c) den Mittelwert aller gleichzeitig gemessenen Signalwerte aller Elektroden (21), insbesondere nur innerhalb desselben Gitters, vom Messwert der betreffenden Elektrode (21) abzuziehen,

d) den, gegebenenfalls gewichteten, Mittelwert aller gleichzeitig gemessenen Signalwerte aller Nachbarelektroden (21u, 21u', 21u") der betreffenden Elektrode (21z), insbesondere nur innerhalb desselben Gitters, vom Messwert der betreffenden Elektrode (21z) abzieht, wobei in einem quadratischen Gitter von Elektroden (21) als Nachbarelektroden (21) insbesondere angesehen werden:

i) die vier unmittelbar an eine Elektrode (21z) angrenzenden Elektroden (21u),

ii) die acht eine Elektrode (21z) umgebenden Elektroden (21u'), wobei gegebenenfalls die einzelnen Nachbarelektroden (21u') mit einem von ihrer Entfernung von der Elektrode abhängigen Gewichtsfaktor gewichtet sind,

iii) diejenigen vier Elektroden (21u") innerhalb eines quadratischen Gitters deren eine Koordinatenposition von der betreffenden Koordinatenposition der Elektrode (21z) um zwei abweicht, deren andere Koordinatenposition mit der betreffenden Koordinatenposition der Elektrode (21z) übereinstimmt.

**10.** Vorrichtung nach einem der vorangehenden Ansprüche, **dadurch gekennzeichnet,**

- **dass** die Analyseeinheit (13) ausgebildet ist, für jede Elektrode (21) laufend abgeleitete Messwerte zu erstellen, wobei innerhalb eines vorgegebenen Zeitraums erstellte Messwerte zu Fenstern ($F_1$, $F_2$, $F_3$), insbesondere mit einer Länge von 20 ms bis 2 Sekunden, zusammengefasst werden und

- **dass** die Analyseeinheit (11) ausgebildet ist, insbesondere mittels FFT oder autoregressiver Modelle, wie vorzugsweise LMS, Recursive Least Square oder Kalmann-Filtern von Ordnung 5 bis 50, die Signalenergie des Signals innerhalb des Fensters ($F_1$, $F_2$, $F_3$) in einem Frequenzbereich mit einer unteren Frequenz von 60 Hz bis 100 Hz und einer oberen Frequenz in einem Frequenzbereich von 150 Hz bis 1 kHz zu ermitteln, und daraus ein Analysesignal (A) zu erstellen, und gegebenenfalls im Rahmen der Basismessung ein Referenzsignal zu erstellen, und

- **dass** gegebenenfalls die Analyseeinheit (13) Frequenzbereiche innerhalb des vorgegebenen Fensters ($F_1$, $F_2$, $F_3$), die in einem Bereich (X) um die Netzfrequenz ($f_N$) oder ein Vielfaches der Netzfrequenz ($2f_N$) liegen, nicht für die Bildung der Signalenergie heranzieht.

**11.** Vorrichtung nach einem der vorangehenden Ansprüche, **dadurch gekennzeichnet, dass** die Steuereinheit (1) dazu ausgebildet ist, einen Stimulus im Bereich einer Elektrode (21), insbesondere mit einem Spannungsstimulus mit einer Frequenz zwischen 1Hz und 100 Hz, abzugeben, und dass die Steuereinheit dazu ausgebildet ist, nach der Abgabe des Stimulus an allen oder einer Anzahl von Elektroden (21)

a) evozierte Potentiale im abgegebenen Signal der jeweiligen Elektrode zu detektieren, oder

b) die Bandleistung des abgegebenen Signals der jeweiligen Elektrode, insbesondere im Bereich zwischen 60Hz und kHz zu detektieren, und

dass die Steuereinheit (1) derart alle Elektroden (21) bzw die von den Elektroden (21) erfassten Gehirnregionen darstellt, in denen aufgrund des Stimulus ein evoziertes Potential oder eine erhöhte Bandleistung im Beriech zwischen 60Hz und 1kHz besteht.

**Claims**

**1.** Apparatus for electrostimulation of a test subject (3), comprising a plurality of electrodes (21) that can be applied to the human brain (31) to trigger specific stimuli on the human brain (31),

- wherein a control unit (1) comprising a stimulation unit (11) is provided, with which electrical stimuli (S) can be applied to the individual or multiple electrodes (21),

- wherein the control unit (1) comprises a measuring unit (12) connected downstream of the electrodes (21) for determining voltages applied to the individual electrodes (21), where appropriate after specification of different mental activities to the test person,

- wherein the control unit (1) comprises an analysis unit (13), which analyses the individual measurement signals detected by measuring electrodes (21),

- wherein the analysis unit (13) is configured, in the context of a preselection, on the basis of the detected measurement signals, to select individual electrodes (21) for the output of a stimulus,
- wherein the analysis unit (13) is configured to examine, during the preselection, the measurement signals (M) at the measurement electrodes, in particular exclusively, for the presence of signal powers or signal energies in the range of 60 Hz to 1 kHz, in particular between 60 Hz and 180 Hz, and
- wherein the control unit (1) comprises a, in particular human-actuatable, selection and actuation unit (14), for selection of one or more electrodes (21) from the electrodes (21) preselected by the analysis unit (13), and for the output by the stimulation unit (11) of a predetermined electrical stimulus (S) to the electrode(s) (21) so selected, which selection and actuation unit is connected downstream of the analysis unit (13) and upstream of the stimulation unit (11),
**characterised in that**
- the control unit is configured to carry out a base measurement by means of the analysis unit (13), during which the test subject (3) carries out a reference activity, and to store the analysis results thereby obtained - associated with the respective measuring electrode (21) - as reference values or reference signals in a reference memory (13a) and to keep them available, and
- the analysis unit (13) comprises a comparison unit, which compares the individual detected analysis values with the stored reference values, wherein the analysis result for each individual electrode (21) indicates by how much the detected analysis values differ from the reference values associated with the respective electrode (21).

2. Apparatus according to claim 1, **characterised in that** the selection and actuation unit (14) comprises a display unit (141) which displays at positions (142) of the display unit (141) the electrodes (21) as well as graphical visualisations of the analysis results (143a, 143b) determined by the analysis unit (13), in particular the preselection results, on the basis of the analysis for the individual electrodes (21).

3. Apparatus according to claim 2, **characterised in that**

- the selection and actuation unit (14) comprises individual selection or actuation elements (144) in the region of the display unit (141),
- the selection or actuation elements (144) are each associated with an electrode (21) and are arranged on the display unit (141) in the region of the position (142) at which the graphical visualisations (143a, 143b) for the electrode (21) concerned are displayed, and
- the selection or actuation elements (144) are configured to select the electrode (21) assigned to them for the output of a stimulus (S) or for the output of a stimulus (S) with the electrode (21) concerned.

4. Apparatus according to any one of the preceding claims, **characterised in that** the selection and actuation unit (14) enables the selection of an electrode (21) preselected by the analysis unit (13) as well as an electrode (21) adjacent thereto, and the stimulation unit (11) is configured to output a stimulus (S) in the form of a current between the two electrodes (21),
wherein in particular the direct current component of the current flowing between the adjacent electrodes (21) as a stimulus (S) is below a predetermined threshold value, preferably

a) **in that** the stimulation unit (11) applies a direct current-free stimulus (S) to the electrodes (21), or
b) **in that** the stimulation unit (11) limits the direct component of the current of the stimulus (S), and wherein in particular the shape of the current of the stimulus is rectangular, triangular or sinusoidal.

5. Apparatus according to any one of the preceding claims, **characterised in that** a detection unit (22) is provided for detecting the reaction of the test subject (3) and is connected to the control unit (1), the detection unit (22) being configured in particular by a microphone for detecting speech of the test subject (3) or by a detector for detecting movements of the test subject (3) or for detecting electro-physiological signals.

6. Apparatus according to any one of the preceding claims, **characterised in that** the stimulation unit (11) is configured to apply stimuli (S) of different, in particular increasing, strength and/or duration to the individual electrodes (21), wherein in particular

a) the stimulation unit (11) is configured to output the individual stimuli (S), in particular in ascending order, when operated manually or automatically, or
b) the control unit (1) controls the stimulation unit (11) to output stimuli (S) in ascending order until the detection unit (22) detects a reaction of the test person (3) or the current limit is reached.

7. Apparatus according to any one of the preceding claims, **characterised in that** the individual electrodes (21) are arranged in a grid or several grids, wherein in particular the electrodes (21) are arranged within the respective grid in a predetermined structure, and/or each of the electrodes (21), except for edge electrodes, has a predetermined number of adjacent electrodes (21) which are arranged at a predetermined position relative to the respective electrode and/or are at the same distance from one another.

8. Apparatus according to any one of the preceding claims, **characterised in that**

   a) the individual electrodes (21) are configured in the same way as one another, and/or
   b) the individual electrodes (21) arranged within a grid are configured in the same way as one another, and/or
   c) that the electrodes (21) are arranged within a grid with a square or hexagonal structure.

9. Apparatus according to any one of the preceding claims, **characterised in that** each of the electrodes (21) is connected downstream of a separate filter (12a, 12b) which is connected upstream of the analysis unit (13) or the measuring unit (12) and which is configured to

   (a) in the presence of a signal energy exceeding or falling below a predetermined threshold or a signal shape deviating from a target shape by more than a predetermined threshold, to suppress the signal concerned and not transmit it to the analysis unit; and/or
   b) to filter out signal components below a cut-off frequency of 1 Hz to 5 Hz, and/or
   c) to subtract the mean value of all simultaneously measured signal values of all electrodes (21), in particular only within the same grid, from the measured value of the electrode (21) concerned,
   d) to subtract the, where appropriate weighted, mean value of all simultaneously measured signal values of all adjacent electrodes (21u, 21u', 21u") of the electrode (21z) concerned, in particular only within the same grid, from the measured value of the electrode (21z) concerned, wherein in a square grid of electrodes (21), adjacent electrodes (21) are in particular considered to be

   i) the four electrodes (21u) directly adjacent to an electrode (21z),
   ii) the eight electrodes (21u') surrounding an electrode (21z), wherein if appropriate the individual adjacent electrodes (21u') are weighted with a weighting factor dependent on their distance from the electrode,
   iii) those four electrodes (21u") within a square grid whose one coordinate position differs from the concerned coordinate position of the electrode (21z) by two, whose other coordinate position coincides with the concerned coordinate position of the electrode (21z).

10. Device according to any one of the preceding claims, **characterised in that**

   - the analysis unit (13) is configured to continuously generate derived measured values for each electrode (21), wherein measured values generated within a predetermined period of time are combined into windows ($F_1$, $F_2$, $F_3$), in particular with a length of 20 ms to 2 seconds, and
   - the analysis unit (11) is configured, in particular by means of FFT or autoregressive models, such as preferably LMS, Recursive Least Square or Kalmann filters of order 5 to 50, to determine the signal energy of the signal within the window ($F_1$, $F_2$, $F_3$) in a frequency range with a lower frequency of 60 Hz to 100 Hz and an upper frequency in a frequency range of 150 Hz to 1 kHz, and to generate an analysis signal (A) therefrom, and where appropriate to generate a reference signal within the framework of the base measurement, and
   - where appropriate, the analysis unit (13) does not use frequency ranges within the predetermined window ($F_1$, $F_2$, $F_3$) which lie in a range (X) around the mains frequency ($f_N$) or a multiple of the mains frequency ($2f_N$) to form the signal energy.

11. Device according to any one of the preceding claims, **characterised in that** the control unit (1) is configured to output a stimulus in the region of an electrode (21), in particular with a voltage stimulus with a frequency between 1 Hz and 100 Hz, and **in that** the control unit is configured, after output of the stimulus to all or a plurality of electrodes (21)

   a) to detect evoked potentials in the output signal of the respective electrode, or
   b) to detect the band power of the emitted signal of the respective electrode, in particular in the range between 60Hz and kHz, and
   **in that** the control unit (1) thus displays all the electrodes (21) or the brain regions detected by the electrodes (21) in which there is an evoked potential or an increased band power in the range between 60 Hz and 1 kHz due to the stimulus.

**Revendications**

1. Dispositif d'électrostimulation d'un sujet (3) comprenant un certain nombre d'électrodes (21) pouvant être appliquées sur le cerveau humain (31) pour déclencher des stimuli électriques spécifiques sur le cerveau humain (31),

   - une unité de commande (1) comprenant une unité de stimulation (11) étant prévue, avec les stimuli électriques (S) pouvant être appliqués au niveau d'une ou plusieurs électrodes (21),
   - l'unité de commande (1) présentant une unité de mesure (12) branchée en aval des électrodes (21) pour déterminer les tensions appliquées aux électrodes (21) individuelles, le cas échéant en fonction de différentes activités intellectuelles réalisées sur les sujets,
   - l'unité de commande (1) présentant une unité d'analyse (13), qui analyse chaque signal de mesure enregistré avec des électrodes de mesure (21),
   - l'unité d'analyse (13) étant conçue pour sélectionner dans le cadre d'une présélection en fonction de l'analyse des signaux de mesure enregistrés certaines des électrodes (21) pour délivrer un stimulus,
   - l'unité d'analyse (13) étant conçue pour étudier lors de la présélection les signaux de mesure (M) au niveau des électrodes de mesure, en particulier exclusivement, concernant l'existence de puissances de signal ou d'énergies de signal se situant dans la plage allant de 60 Hz à 1 kHz, en particulier comprises entre 60 Hz et 180 Hz, et
   - l'unité de commande (1) présentant une unité de sélection et d'actionnement (14) actionnable par l'Homme pour sélectionner une ou plusieurs électrodes (21) parmi les électrodes (21) présélectionnées par l'unité d'analyse (13), ainsi que pour délivrer un stimulus (S) électrique prédéfini à(aux) l'électrode(s) (21) ainsi présélectionnée(s) par l'unité de stimulation (11), qui est branchée en aval de l'unité d'analyse (13) et qui branchée en amont de l'unité de stimulation (11),
   **caractérisé**
   - **en ce que** l'unité de commande est conçue de manière à réaliser au moyen d'une unité d'analyse (13) une mesure de base, lors de laquelle le sujet (3) effectue une activité de référence, et les résultats d'analyses ainsi obtenus, associés aux électrodes (21) de mesure respectives, sont enregistrés et gardés à disposition en tant que valeurs de référence ou signal de référence dans une mémoire de référence (13a), et
   - **en ce que** l'unité d'analyse (13) présente une unité de comparaison, qui compare les valeurs d'analyse déterminées aux valeurs de référence enregistrées, le résultat d'analyse indique pour chaque électrode (21) individuelle, comment les valeurs d'analyse déterminées se distinguent des valeurs de référence associés aux électrodes (21) respectives.

2. Dispositif selon la revendication 1, **caractérisé en ce que** l'unité de sélection et d'actionnement (14) présente une unité d'affichage (141), qui représente les électrodes (21), ainsi que les résultats d'analyse déterminés par l'unité d'analyse (13), en particulier les résultats de présélection, sur la base de l'analyse pour les électrodes (21) individuelles aux positions (142) des visualisations graphiques (143a, 143b) des résultats d'analyse de l'unité d'affichage (141).

3. Dispositif selon la revendication 2, **caractérisé**

   - **en ce que** l'unité de sélection et d'actionnement (14) présente dans la zone de l'unité d'affichage (141) des éléments de sélection et d'actionnement (144) individuels,
   - **en ce que** les éléments de sélection ou d'actionnement (144) sont associés respectivement à une électrode (21) et sont disposés sur l'unité d'affichage (141) dans la zone de la position (142), au niveau de laquelle les visualisations graphiques (143a, 143b) sont représentées pour l'électrode (21) concernée, et
   - **en ce que** les éléments de sélection ou d'actionnement (144) sont conçus pour la sélection de l'électrode (21) associée à ceux-ci pour la délivrance d'un stimulus (S) ou pour la délivrance d'un stimulus (S) avec l'électrode (21) concernée.

4. Dispositif selon l'une quelconque des revendications précédentes, **caractérisé en ce que** l'unité de sélection et d'actionnement (14) permet la sélection d'une des électrodes (21) présélectionnées par l'unité d'analyse (13), ainsi que l'une des électrodes (21) adjacentes à celle-ci, et l'unité de stimulation (11) est conçue pour délivrer un stimulus (S) sous la forme d'un courant entre les deux électrodes (21),
   en particulier la composante en courant continu du courant circulant entre les électrodes (21) adjacentes en tant que stimulus (S) se situant en dessous d'une valeur seuil prédéfinie, de préférence

> a) l'unité de stimulation (11) appliquant un stimulus (S) sans courant continu sur les électrodes (21), ou
> b) l'unité de stimulation (11) limitant la composante continue du courant du stimulus (S), et en particulier l'allure du courant du stimulus présentant une allure rectangulaire, triangulaire ou sinusoïdale.

**5.** Dispositif selon l'une quelconque des revendications précédentes, **caractérisé en ce qu'**une unité d'enregistrement (22) est prévue pour l'enregistrement de la réaction du sujet (3) et est raccordée à l'unité de commande (1), l'unité d'enregistrement (22) étant conçue en particulier par un microphone pour l'enregistrement de la voix du sujet (3) ou par un détecteur pour l'enregistrement de mouvements du sujet (3) ou pour l'enregistrement de signaux électrophysiologiques.

**6.** Dispositif selon l'une quelconque des revendications précédentes, **caractérisé en ce que** l'unité de stimulation (11) est conçue pour appliquer des stimuli (S) d'intensité et/ou de durée différente(s), en particulier croissante(s), sur les électrodes individuelles (21), en particulier,

> a) l'unité de stimulation (11) étant conçue pour délivrer les stimuli (S) individuels, en particulier selon un ordre croissant, par actionnement manuel ou automatique, ou
> b) l'unité de commande (1) commande l'unité de stimulation (11) pour délivrer des stimuli (S) selon un ordre croissant, jusqu'à ce que l'unité d'enregistrement (22) détecte une réaction du sujet (3) ou que la limitation de courant soit atteinte.

**7.** Dispositif selon l'une quelconque des revendications précédentes, **caractérisé en ce que** les électrodes (21) individuelles sont disposées dans une grille ou plusieurs grilles, en particulier les électrodes (21) étant disposées à l'intérieur de la grille respective selon une structure prédéfinie, et/ou **en ce que** chacune des électrodes (21) présentant jusqu'aux électrodes périphériques un nombre prédéfini d'électrodes voisines (21), qui sont disposées par rapport à l'électrode respective au niveau d'une position prédéterminée et/ou qui sont équidistantes les unes des autres.

**8.** Dispositif selon l'une quelconque des revendications précédentes, **caractérisé en ce que**

> a) les électrodes (21) individuelles sont conçues de manière similaire entre elles, et/ou
> b) les électrodes (21) individuelles disposées à l'intérieur d'une grille sont conçues de manière similaire, et/ou
> c) **en ce que** les électrodes (21) sont disposées à l'intérieur d'une grille selon une structure quadratique ou hexagonale.

**9.** Dispositif selon l'une quelconque des revendications précédentes, **caractérisé en ce que** chacune des électrodes (21) est branchée en aval d'un filtre (12a, 12b) distinct, qui est branché en amont de l'unité d'analyse (13) ou de l'unité de mesure (12) et qui est conçue pour

> a) supprimer en présence d'une énergie de signal étant supérieure ou inférieure à une valeur seuil prédéfinie ou d'une forme de signal différente d'une forme nominale supérieure à une valeur seuil prédéfinie le signal concerné et ne pas le transmettre à l'unité d'analyse, et/ou
> b) filtrer des composantes de signal inférieures à une fréquence limite allant de 1 Hz à 5 Hz, et/ou
> c) soustraire la valeur moyenne de toutes les valeurs de signaux mesurées simultanément de toutes les électrodes (21), en particulier uniquement à l'intérieur de la même grille, de la valeur mesurée de l'électrode (21) concernée,
> d) soustraire la valeur moyenne, le cas échéant pondérée, de toutes les valeurs de signaux mesurées simultanément de toutes les électrodes voisines (21u, 21u', 21u") de l'électrode concernée (21z), en particulier uniquement à l'intérieur de la même grille, de la valeur de mesurée de l'électrode (21z) concernée, dans une grille quadratique d'électrodes (21) sont considérées en particulier comme électrodes voisines (21) :

>> i) les quatre électrodes (21u) directement adjacentes à une électrode (21z),
>> ii) les huit électrodes (21u') avoisinantes une électrode (21z), le cas échéant les électrodes voisines (21u') individuelles étant pondérées avec un facteur de pondération dépendant de son retrait de l'électrode,
>> iii) lesdites quatre électrodes (21u") à l'intérieur d'une grille quadratique dont une position de coordonnées s'écarte de deux de la position de coordonnées concernée de l'électrode (21z), dont l'autre position de coordonnées coïncide avec la position de coordonnées concernée de l'électrode (21z).

**10.** Dispositif selon l'une quelconque des revendications précédentes, **caractérisé**

- **en ce que** l'unité d'analyse (13) est conçue pour établir pour chaque électrode (21) des valeurs de mesure dérivées en continu, à l'intérieur d'une période prédéfinie des valeurs de mesure établies par rapport à des fenêtres ($F_1$, $F_2$, $F_3$), en particulier avec une durée allant de 20 ms à 2 secondes, sont regroupées et
- **en ce que** l'unité d'analyse (11) est conçue, en particulier au moyen de FFT ou de modèles autorégressifs, comme de préférence LMS, l'algorithme des moindres carrés récursifs (*Recursive Least Square*) ou les filtres de Kalman de l'ordre 5 à 50, pour déterminer l'énergie de signal du signal à l'intérieur de la fenêtre ($F_1$, $F_2$, $F_3$) dans une plage de fréquences présentant une fréquence inférieure allant de 60 Hz à 100 Hz et une fréquence supérieure dans une plage de fréquences allant de 150 Hz à 1 kHz, et ainsi établir un signal d'analyse (A), et le cas échéant établir un signal de référence dans le cadre de la mesure de base, et
- **en ce que** le cas échéant l'unité d'analyse (13) n'utilise pas pour la formation de l'énergie de signal les plages de fréquence à l'intérieur de la fenêtre prédéfinie ($F_1$, $F_2$, $F_3$), qui se situent dans une plage (X) autour de la fréquence de réseau ($f_N$) ou d'un multiple de la fréquence de réseau ($2f_N$).

**11.** Dispositif selon l'une quelconque des revendications précédentes, **caractérisé en ce que** l'unité de commande (1) est conçue pour délivrer un stimulus dans la zone d'une électrode (21), en particulier avec un stimulus de tension présentant une fréquence comprise entre 1 Hz et 100 Hz, et **en ce que** l'unité de commande est conçue pour, après la délivrance du stimulus à tous ou à un certain nombre d'électrodes (21)

a) détecter des potentiels évoqués dans le signal délivré de l'électrode respective, ou
b) détecter la puissance de bande du signal délivré de l'électrode respective, en particulier dans la plage comprise entre 60 Hz et kHz, et

**en ce que** l'unité de commande (1) représente toutes les électrodes (21) ou les régions du cerveau enregistrées par les électrodes (21), de telle sorte qu'il existe dans celles-ci grâce au stimulus un potentiel évoqué ou une puissance de bande accrue dans la plage comprise entre 60 Hz et 1 kHz.

Fig. 1

Fig. 8

Fig. 2

Fig. 3

Fig. 4

Fig. 5

M

F1    F2    F3    t

# Fig. 6

x

80   95   105        180   f [Hz]

$f_N$        $2f_N$

# Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2011264165 A1
- US 2012253421 A1